# EUROPEAN PATENT SPECIFICATION

(11) **EP 1 414 800 B1**
(45) Date of publication and mention of the grant of the patent: **29.12.2004**
(21) Application number: 01952573.2
(22) Date of filing: 11.07.2001
(51) Int. Cl.: C07D 213/38

(54) **A NOVEL SYNTHESIS FOR HETEROARYLAMINE COMPOUNDS**
Neue Synthese für Heteroarylamin-Verbindungen
SYNTHESE DE COMPOSES HETEROARYLAMINE

(43) Date of publication of application: 06.05.2004
(73) Proprietor: BOEHRINGER INGELHEIM PHARMACEUTICALS INC., Ridgefield, Connecticut 06877-0368 (US)
(72) Inventor: YEE, Nathan, Danbury, CT 06811 (US); KAPADIA, Suresh, R., Danbury, CT 06810 (US); SONG, Jinhua, J., Hopewell Junction, NY 12533 (US)
(74) Representative: Hammann, Heinz, Dr.
(86) International application number: PCT/US2001/021671
(87) International publication number: WO 2003/006431

(56) References cited:
- WO-A-00/55139
- US-A- 5 502 059

## Description

### FIELD OF INVENTION:

This invention relates to the synthesis of heteroarylamine compounds which are useful in the production of heteroaryl ureas a key component in pharmaceutically active compounds possessing a heteroaryl urea group.

### BACKGROUND OF THE INVENTION

Aryl- and heteroaryl-substituted ureas have been described as inhibitors of cytokine production. These inhibitors are described as effective therapeutics in cytokine-mediated diseases, including inflammatory and autoimmune diseases. Examples of such compounds are reported in WO 99/23091 and in WO 98/52558.

A key step in the synthesis of these compounds is the formation of the urea bond. Various methods have been reported to accomplish this. For example, as reported in the above references, an aromatic or heteroaromatic amine, Ar₁NH₂, may be reacted with an aromatic or heteroaromatic isocyanate, Ar₂NCO, to generate the urea Ar₁NHC(O)NHAr₂.

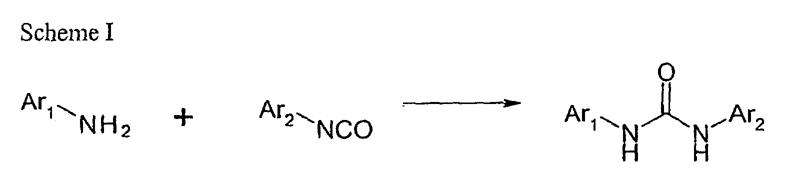

If not commercially available, one may prepare the isocyanate by reaction of an aryl or heteroaryl amine Ar₂-NH₂ with phosgene or a phosgene equivalent, such as bis(trichloromethyl) carbonate (triphosgene) (P. Majer and R. S. Randad, J. Org. Chem. 1994, 59, 1937) or trichloromethyl chloroformate (diphosgene) (K. Kurita, T. Matsumura and Y. Iwakura, J. Org. Chem. 1976, 41, 2070) to form the isocyanate Ar₂-NCO, followed by reaction with Ar₁NH₂ to provide the urea. Other approaches to forming the urea reported in the chemical literature include reaction of a carbamate with an aryl or heteroaryl amine, (see for example B. Thavonekham, Synthesis, 1997, 1189 and T. Patonay et al., Synthetic Communications, 1996, 26, 4253) as shown in Scheme U. US Provisional Application No. 60/143,094 also discloses a process of making heteroaryl ureas by reacting particular carbamate intermediates with the desired arylamine.

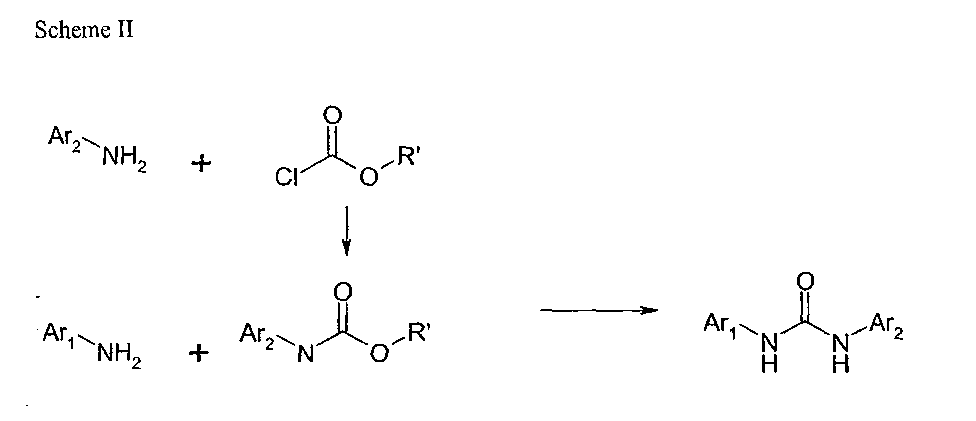

U.S. Application no. 09/505,582 and PCT/US00/03865 describe cytokine inhibiting ureas of the following formula :

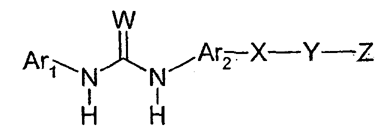

An intermediate required to prepare preferred compounds described therein has a 1,4-disubstituted naphthalene as Ar₂ and is illustrated in the formula below.

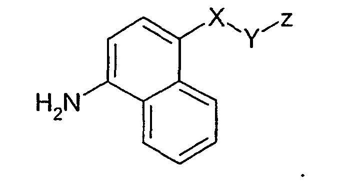

The preparation of these intermediates require the coupling of the naphthyl ring with X. Preferred X include aryl and heteroaryl groups. Previously described methods, including U.S. Application No. 09/505,582 and PCT/US00/03865 achieve the coupling of these aromatic residues by using a coupling reaction catalyzed by a transition metal, such as palladium, in the presence of a ligand, such as triphenyl phosphine. Coupling methods include Stille coupling, requiring the preparation of a tributylstannyl intermediate, or a Suzuki coupling, requiring the preparation of a boronic acid intermediate (Scheme III).

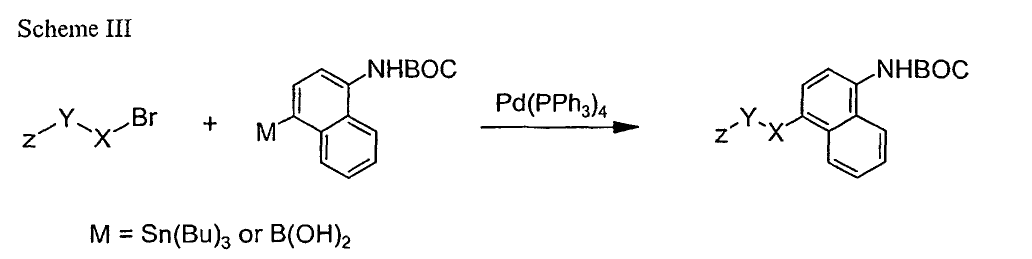

Some steps in these methods require cooling to extreme temperatures (-78 °C). Others require reaction under high pressure, require chromatography to purify the product, or use expensive reagents. For these reasons, these methods are not suitable for large-scale or industrial-scale production.

### SUMMARY OF THE INVENTION

It is an object of the invention to provide a novel method of producing heteroaryl amines of the formula(I) : 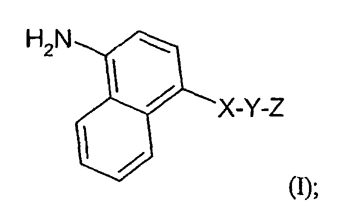 wherein X, Y and Z are described below, the heteroarylamines are useful in the production of heteroaryl ureas as mentioned above.

### DETAILED DESCRIPTION OF THE INVENTION

Disclosed herein is a novel process for preparing preferred heteroarylamine intermediates including those heteroarylamine intermediates described in U.S. Application No. 09/505,582, and PCT/US00/03865. The processes described herein have several advantages. They use inexpensive starting materials and reagents, the reactions are run at moderate temperatures, there are no high-pressure reactions and chromatography is not required.

The novel feature of the invention is the construction of naphthalene ring, as exemplified in Scheme I below, from the appropriately substituted carboxylic acid **5,** which in turn was synthesized beginning from a novel ester of the formula (II) and a diester such as diethyl succinate. Any of the compounds of the formula (II) as described herein can be synthesized from readily available and cost efficient starting materials such as example 1 below.

This invention provides a novel strategy for the synthesis of heteroarylamine compounds of the formula (I): 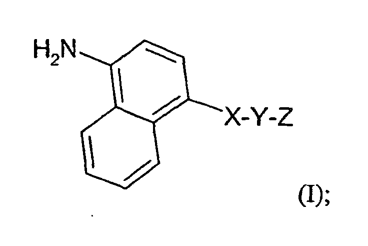 wherein:
the naphthyl ring is further optionally substituted by one or more R₁ or R₂;
X is chosen from
   a C₅₋₈ cycloalkyl and cycloalkenyl optionally substituted with one to two oxo groups or one to three C₁₋₄ alkyl, C₁₋₄ alkoxy or C₁₋₄ alkylamino chains each being branched or unbranched;
aryl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl and pyrazinyl; each being optionally independently substituted with one to three C₁₋₄alkyl, C₁₋₄alkoxy, hydroxy, nitro, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
Y is chosen from
   a bond and a C₁₋₄ saturated or unsaturated branched or unbranched carbon chain optionally partially or fully halogenated, wherein one or more methylene groups are optionally replaced by O, N, or S(O)ₘ and wherein Y is optionally independently substituted with one to two oxo groups, phenyl or one or more C₁₋₄ alkyl optionally substituted by one or more halogen atoms;
Z is chosen from .
   phenyl, pyridinyl, pyrimidinyl, pyridazinyl, pyrazinyl, imidazolyl, pyrazolyl, triazolyl, tetrazolyl, furanyl, thienyl, pyranyl, each being optionally substituted with one to three halogen, C₁₋₆ alkyl, C₁₋₆ alkoxy, hydroxy, amino, mono- or di-(C₁₋₃ alkyl)amino, C₁₋₆ alkyl-S(O)ₘ, CN, CONH₂, COOH or phenylamino wherein the phenyl ring is optionally substituted with one to two halogen, C₁₋₆ alkyl or C₁₋₆ alkoxy;
   tetrahydropyranyl, tetrahydrofuranyl, 1,3-dioxolanonyl, 1,3-dioxanonyl, 1,4-dioxanyl, morpholinyl, thiomorpholinyl, thiomorpholinyl sulfoxidyl, thiomorpholinyl sulfonyl, piperidinyl, piperidinonyl, piperazinyl, tetrahydropyrimidonyl, cyclohexanonyl, cyclohexanolyl, pentamethylene sulfidyl, pentamethylene sulfoxidyl, pentamethylene sulfonyl, tetramethylene sulfide, tetramethylene sulfoxidyl or tetramethylene sulfonyl each being optionally substituted with one to three nitrile, C₁₋₆ alkyl, C₁₋₆ alkoxy, hydroxy, amino, mono- or di-(C₁₋₃ alkyl)amino-C₁₋₃ alkyl, CONH₂, phenylamino-C₁₋₃ alkyl or C₁₋₃ alkoxy-C₁₋₃ alkyl;
   halogen, C₁₋₄ alkyl, nitrile, amino, hydroxy, C₁₋₆ alkoxy, NH₂C(O), mono- or di(C₁₋₃alkyl) aminocarbonyl, mono- or di(C₁₋₃alkyl)amino, secondary or tertiary amine wherein the amino nitrogen is covalently bonded to C₁₋₃ alkyl or C₁₋₅ alkoxyalkyl, pyridinyl-C₁₋₃ alkyl, imidazolyl-C₁₋₃ alkyl, tetrahydrofuranyl-C₁₋₃ alkyl, nitrile-C₁₋₃ alkyl, carboxamide-C₁₋₃ alkyl, phenyl, wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, or phenyl-S(O)ₘ, wherein the phenyl ring is optionally substituted with one to two halogen, C₁₋₆ alkoxy, hydroxy, halogen or mono- or di-(C₁₋₃ alkyl)amino;
   C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
R₁ and R₂ are independently chosen from:
   a C₁₋₆ branched or unbranched alkyl optionally partially or fully halogenated, C₁₋₄ branched or unbranched alkoxy, each being optionally partially or fully halogenated, halogen, C₁₋₃ alkyl-S(O)ₘ optionally partially or fully halogenated and phenylsulfonyl; and
   m is 0, 1 or 2.

The process of the invention in its broadest generic aspect is provided below and exemplified in a non-limiting embodiment shown in Scheme 1:
said process comprising:
   a) reacting a Z-Y-X-COO-Rₓ ester (II) wherein Rₓ is C₁₋₅alkyl or aryl with a di-alkyl or diaryl ester (III) in a suitable solvent protic or aprotic, polar or nonpolar, preferably aprotic such as THF, DME, DMSO, ether, dioxane, CH₂Cl₂, CHCl₃, toluene, pyridine or DMF, or suitable alcohols, preferably the solvent is chosen from THF and DMSO, more preferably THF, and a suitable base such as organic or inorganic bases such as NaH, NaNH₂, sodium alkoxides such as Na-t-butoxide, Na-ethoxide, NaOH, pyridine, TEA, DBU or BuLi, preferably NaH or Na t-butoxide, and optionally where appropriate as in Example 3, in the presence of an additive such as DMPU and HMPA, preferably DMPU, under the temperature of about 0 to 200°C for a reaction time of about 5 min to 24 h, preferably when using the preferred solvent THF at 60-70 °C for about 8 h and isolating the compound intermediate (IV). Examples 1 & 2 are representative methods for preparation of compounds of the formula(II), methods of preparing other compounds of the formula(II) is within the skill in the art. 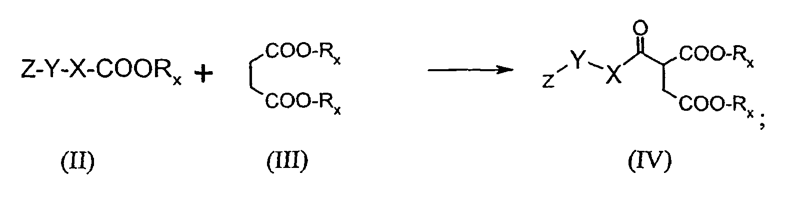
   b) subjecting the product of step a) to acidic or basic hydrolysis, preferably acidic hydrolysis, and decarboxylation under suitable acid conditions apparent to those skilled in the art, such as conc. H₂SO₄ in HOAc at a temperature of about 50 to 200 °C and for about 5 min to 24 hours, preferably about 100 °C for about 7 h; followed by esterification under appropriate conditions with a C₁₋₅alcohol, preferably EtOH; subsequent phenyl nucleophilic addition via for example a phenyl Grignard reagent PhMgBr, phenylLi, phenylZnCl, preferably phenyl Grignard, the phenyl being optionally substituted by R₁ and/or R₂; reductive cleavage under appropriate conditions such as HCOONH₄ / Pd/C / EtOH to form a carboxylic acid compound which on treatment with a strong mineral acid such as H₂SO₄, HCl, MeSO₃H, CF₃SO₃H, PPA or Lewis acid such as SnCl₄, AlCl₃, BF₃-OEt₂ and Yb(OTf)₂, preferably PPA, optionally in a suitable solvent at RT to 200°C, preferably about 110°C, to form a product intermediate of the formula(V), and isolating the product: 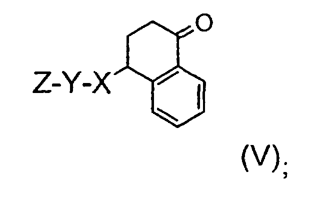
   c) reacting the product from step b) with HNR_{y}R_{z} or it's respective salt thereof, to form an enamine or imine, preferably an oxime, compound of the formula(VI) under suitable conditions. 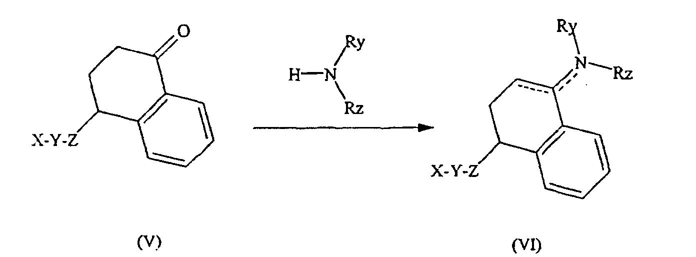
wherein R_{y} is C₁₋₅alkyl or hydrogen, R_{z} is C₁₋₅alkyl, hydrogen or OH with the proviso that when formula (VI) is an enamine tautomer then R_{y} and R_{z} are both C₁₋₅alkyl, or when formula (VI) is an imine tautomer then R_{z} is OH, C₁₋₅alkyl or hydrogen and R_{y} is not present:

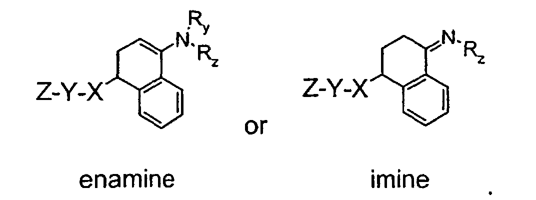

In a preferred but nonlimiting embodiment, forming an oxime by adding NH₂OH·HCl (where R_{y} is H and R_{z} is OH) in a suitable solvent such as-EtOH with a suitable base such as NaOH at about RT for about 1 to 24 h, preferably 18 h;
c) 1) where the product of step c) is an imine, preferably an oxime (R_{y} = H, R_{z} = OR), preferably in a one pot reaction acylating and reducing the product of step c) under conditions known in the art, a preferred but non-limiting example is acetylating/reducing conditions, such as treating compound (VI) with acetic anhydride, acetic acid and a suitable reducing agent such as Fe, SnCl₂ and Zn, preferably Fe, at about 55°C for about 5 hours; then treating the unsaturated amide product (**8**) under oxidizing conditions capable of forming the naphthalene ring of the formula(I) above, for example, treating the amide product(**8**) with an oxidizing reagant such as DDQ, O₂, CrO₃ and KMnO₄, preferably DDQ, in a nonpolar solvent such as methylene chloride, at about 0 to 50 °C, preferably RT for about 0.5 to 10 h, preferably 5 h; followed by deprotection by methods known in the art to provide the formation of formula (I): 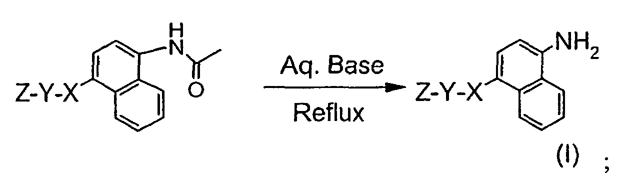 or
2) where the product of step c) is an enamine, oxidizing the enamine under suitable oxidizing conditions to form the naphthalene ring, then deprotecting the nitrogen to form the amine of the formula (I). In a non-limiting example, R_{y} and R_{z} are benzyl, oxidation to the naphthalene ring may be accomplished as described above, and debenzylation may be accomplished by methods known to those skilled in the art, for example H₂/palladium/C.

Compounds of the formula (I) possessing a particularly desired Ar-X-Y-Z combination can be synthesized without undue experimentation by variations apparent to those of ordinary skill in the art in view of the teachings in this specification and the state of the art. More specific examples of possible X-Y-Z combinations are to be found in PCT application no. PCT/US00/03865 and US application no.09/505,582.

In another embodiment of the invention there is provided a novel process of making compounds of the fonnula(I) as described above and wherein:
X is chosen from
   a C₅₋₈ cycloalkyl and cycloalkenyl optionally substituted with one to two oxo groups or one to three C₁₋₄ alkyl, C₁₋₄ alkoxy or C₁₋₄ alkylamino chains each being branched or unbranched;
   aryl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl and pyrazinyl; each being optionally independently substituted with one to three C₁₋₄ alkyl, nitro, nitrile, mono- or di-(C₁₋₃alkyl)amino, mono- or di-(C₁₋₃ alkylamino)carbonyl, NH₂C(O), C₁₋₆ alkyl-S(O)ₘ or halogen;
Y is chosen from
   a bond and
   a C₁₋₄ saturated or unsaturated carbon chain wherein one of the carbon atoms is optionally replaced by O, N, or S(O)ₘ and wherein Y is optionally independently substituted with one to two oxo groups, phenyl or one or more C₁₋₄ alkyl optionally substituted by one or more halogen atoms;
Z is chosen from:
   phenyl, pyridinyl, pyrimidinyl, pyridazinyl, pyrazinyl, imidazolyl, furanyl, thienyl, dihydrothiazolyl, dihydrothiazolyl sulfoxidyl, pyranyl, pyrrolidinyl which are optionally substituted with one to three nitrile, C₁₋₃ alkyl, C₁₋₃ alkoxy, hydroxy, amino, mono- or di- (C₁₋₃ alkyl)amino or CONH₂;
   tetrahydropyranyl, tetrahydrofuranyl, 1,3-dioxolanonyl, 1,3-dioxanonyl, 1,4-dioxanyl, morpholinyl, thiomorpholinyl, thiomorpholinyl sulfoxidyl, piperidinyl, piperidinonyl, piperazinyl, tetrahydropyrimidonyl, pentamethylene sulfidyl, pentamethylene sulfoxidyl, pentamethylene sulfonyl, tetramethylene sulfidyl, tetramethylene sulfoxidyl or tetramethylene sulfonyl which are optionally substituted with one to three nitrile, C₁₋₃ alkyl, C₁₋₃ alkoxy, hydroxy, amino, mono- or di-(C₁₋₃ alkyl)amino or CONH₂;
   nitrile, C₁₋₆ alkyl-S(O)ₘ, halogen, hydroxy, C₁₋₄ alkoxy, amino, mono- or di-(C₁₋₆ alkyl)amino, mono- or di-(C₁₋₃ alkyl)aminocarbonyl and NH₂C(O).

In yet another embodiment of the invention there is provided a novel process of making compounds of the formula(I) as described immediately above and wherein:
X is chosen from
   aryl, pyridinyl, pyrimidinyl, benzimidazole, 3H-imidazo[4,5-b]pyridine, piperazinyl, pyridazinyl and pyrazinyl; each being optionally independently substituted with one to three C₁₋₄ alkyl, nitro, nitrile, mono- or di-(C₁₋₃ alkyl)amino, mono- or di-(C₁₋₃ alkylamino)carbonyl, NH₂C(O), C₁₋₆ alkyl-S(O)ₘ or halogen;
Y is chosen from
   a bond and
   a C₁₋₄ saturated carbon chain wherein one of the carbon atoms is optionally replaced by O, N or S and wherein Y is optionally independently substituted with an oxo group;
Z is chosen from
   phenyl, pyridinyl, pyrimidinyl, pyridazinyl, pyrazinyl, imidazolyl, dihydrothiazolyl, dihydrothiazolyl sulfoxide, pyranyl and pyrrolidinyl which are optionally substituted with one to two C₁₋₂ alkyl or C₁₋₂ alkoxy;
   tetrahydropyranyl, morpholinyl, thiomorpholinyl, thiomorpholinyl sulfoxidyl, piperidinyl, piperidinonyl, piperazinyl and tetrabydropyrimidonyl which are optionally substituted with one to two C₁₋₂ alkyl or C₁₋₂ alkoxy; and C₁₋₃ alkoxy.

In yet still another embodiment of the invention there is provided a novel process of making compounds of the formula(I) as described immediately above and wherein:
X is chosen from
   pyridinyl and pyrimidinyl, each being optionally independently substituted with one to three C₁₋₄ alkyl, nitro, nitrile, mono- or di-(C₁₋₃ alk-yl)amino, mono- or di-(C₁₋₃ alkylamino)carbonyl, NH₂C(O), C₁₋₆ alkyl-S(O)ₘ or halogen;
Y is chosen from
   a bond,
   -CH₂-, -CH₂CH₂-, -C(O)-, -O-, -S-, -NH-CH₂CH₂CH₂-, -N(CH₃)- and -NH-;

In yet a further embodiment of the invention there is provided a novel process of making compounds of the formula(I) as described immediately above and wherein:
Y is chosen from
   -CH₂₋, -NH-CH₂CH₂CH₂- and -NH- and
Z is morpholinyl.

All terms as used herein in this specification, unless otherwise stated, shall be understood in their ordinary meaning as known in the art. For example, "C₁₋₆alkoxy" is a C₁₋₆alkyl with a terminal oxygen, such as methoxy, ethoxy, propoxy, pentoxy and hexoxy. All alkyl, alkenyl and alkynyl groups shall be understood as being branched or unbranched where structurally possible and unless otherwise specified. Other more specific definitions are as follows:

The term "aroyl" as used in the present specification shall be understood to mean "benzoyl" or "naphthoyl".

The term "aryl" as used herein shall be understood to mean aromatic carbocycle or heteroaryl as defined herein.

The term "carbocycle" shall be understood to mean an aliphatic hydrocarbon radical containing from three to twelve carbon atoms. Carbocycles include hydrocarbon rings containing from three to ten carbon atoms. These carbocycles may be either aromatic and non-aromatic ring systems. The non-aromatic ring systems may be mono- or polyunsaturated. Preferred carbocycles include but are not limited to cyclopropyl, cyclobutyl, cyclopentyl, cyclopentenyl, cyclohexyl, cyclohexenyl, cycloheptanyl, cycloheptenyl, phenyl, indanyl, indenyl, benzocyclobutanyl, dihydronaphthyl, tetrahydronaphthyl, naphthyl, decahydronaphthyl, benzocycloheptanyl and benzocycloheptenyl. Certain terms for cycloalkyl such as cyclobutanyl and cyclobutyl shall be used interchangeably.

The term "heterocycle", unless otherwise noted, refers to a stable nonaromatic 4-8 membered (but preferably, 5 or 6 membered) monocyclic or nonaromatic 8-11 membered bicyclic heterocycle radical which may be either saturated or unsaturated. Each heterocycle consists of carbon atoms and one or more, preferably from 1 to 4 heteroatoms selected from nitrogen, oxygen and sulfur. The heterocycle may be attached by any atom of the cycle, which results in the creation of a stable structure. Unless otherwise stated, heterocycles include but are not limited to, for example oxetanyl, pyrrolidinyl, tetrahydrofuranyl, tetrahydrothiophenyl, piperidinyl, piperazinyl, morpholinyl, tetrahydropyranyl, dioxanyl, tetramethylene sulfonyl, tetramethylene sulfoxidyl, oxazolinyl, thiazolinyl, imidazolinyl, tertrahydropyridinyl, homopiperidinyl, pyrrolinyl, tetrahydropyrimidinyl, decahydroquinolinyl, decahydroisoquinolinyl, thiomorpholinyl, thiazolidinyl, dihydrooxazinyl, dihydropyranyl, oxocanyl, heptacanyl, thioxanyl, dithianyl or 2-oxa- or 2-thia-5-aza-bicyclo[2.2.1]heptanyl.

The term "heteroaryl", unless otherwise noted, shall be understood to mean an aromatic 5-8 membered monocyclic or 8-11 membered bicyclic ring containing 1-4 heteroatoms such as N,O and S. Unless otherwise stated, such heteroaryls include: pyridinyl, pyridonyl, quinolinyl, dihydroquinolinyl, tetrahydroquinoyl, isoquinolinyl, tetrahydroisoquinoyl, pyridazinyl, pyrimidinyl, pyrazinyl, benzimidazolyl, benzthiazolyl, benzoxazolyl, benzofuranyl, benzothiophenyl, benzpyrazolyl, dihydrobenzofuranyl, dihydrobenzothiophenyl, benzooxazolonyl, benzo[1,4]oxazin-3-onyl, benzodioxolyl, benzo[1,3]dioxol-2-onyl, tetrahydrobenzopyranyl, indolyl, indolinyl, indolonyl, indolinonyl, phthalimidyl.

Terms which are analogs of the above cyclic moieties such as aryloxy or heteroaryl amine shall be understood to mean an aryl, heteroaryl, heterocycle as defined above attached to it's respective functional group.

As used herein, "nitrogen" and "sulfur" include any oxidized form of nitrogen and sulfur and the quatemized form of any basic nitrogen.

The term "halogen" as used in the present specification shall be understood to mean bromine, chlorine, fluorine or iodine except as otherwise noted.
DDQ - 2,3-Dichloro-5,6-dicyano-1,4-benzoquinone;
PPA - Polyphosphoric acid;
HOAc - acetic acid;
RT or rt - room temperature;
n-BuLi - n-Butyllithium
DME - 1,2-Dimethoxyethane
DMSO - Methyl sulfoxide
DMF - *N*,*N*-Dimethylformamide
DBU - 1,8-Diazabicyclo[5.4.0]undee-7-ene
DMPU - *N*,*N*'-Dimethylpropyleneurea
HMPA - Hexamethylphosphoramide
TEA - Triethylamine
THF - Tetrahydrofuran.

The compounds of the invention are only those which are contemplated to be 'chemically stable' as will be appreciated by those skilled in the art. For example, a compound which would have a 'dangling valency', or a 'carbanion' are not compounds contemplated by the invention.

In order that this invention be more fully understood, the following examples are set forth in the overall reaction scheme below. These examples are for the purpose of illustrating preferred embodiments of this invention, and are not to be construed as limiting the scope of the invention in any way. Sample methods and starting materials to make compound (1) in Scheme I are shown in Examples 1 and 2 below.

### EXAMPLES

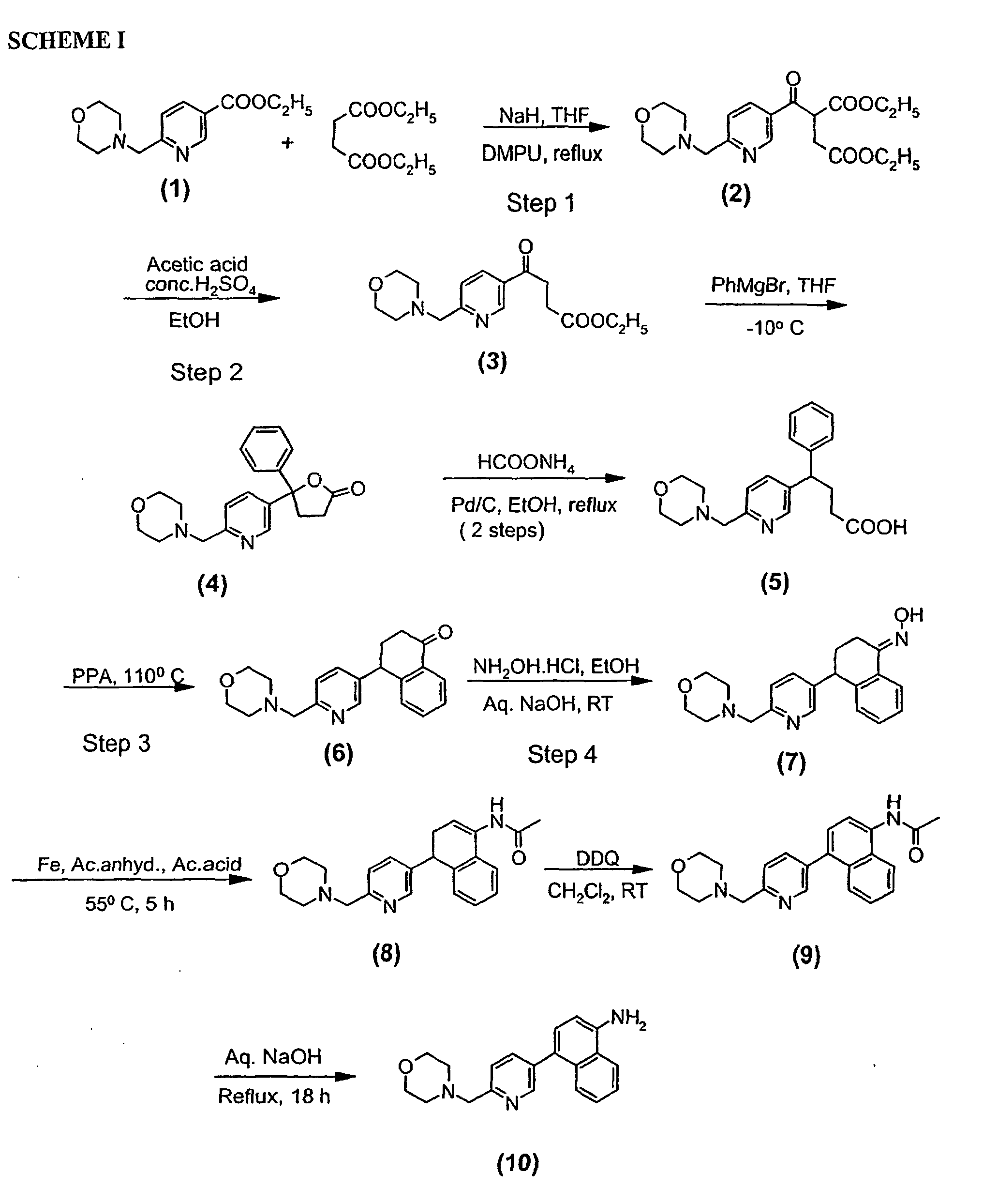

### Example 1

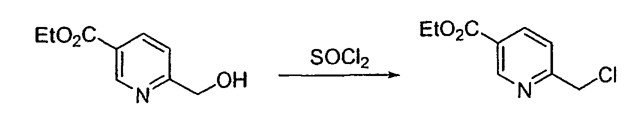

**Preparation of chloride:** A solution of alcohol shown above (18.0 g, 100 mmol) in 200 mL of CH₂Cl₂ was prepared and cooled in an ice-water bath. A solution of SOCl₂ (22 mL, 300 mmol) in 100 mL of CH₂Cl₂ was added to the above solution at the rate to keep the internal temperature below 10°C. After the addition, the cooling bath was removed and the reaction mixture was warmed to room temperature over 2 h. The reaction mixture was evaporated to remove all volatile by rotavap. The residue was dissolved in 150 mL of CH₂Cl₂ and saturated sodium bicarbonate solution was added until pH = 9. The aqueous layer was extracted with CH₂Cl₂. The combined organic layers were dried (MgSO₄) and concentrated to give 20.0 g (100%) of the desired chloride. ¹H NMR (CDCL₃): δ 9.15 (s, 1H), 8.33 (d, J = 8 Hz, 1H), 7.58 (d, J = 8 Hz), 4.73 (s, 2H), 4.42 (q, J = 7 Hz, 2H), 1.42 (t, J = 7 Hz, 3H).

### Example 2

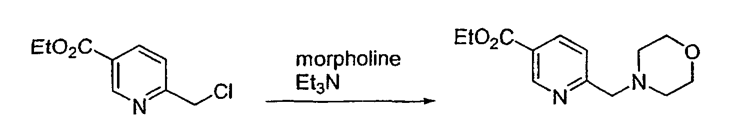

**Preparation of morpholine moiety:** A solution of chloride from Example 1 above (20 g, 100 mmol) and triethylamine (15.2 g, 150 mmol) in 125 mL of CH₂Cl₂ was prepared. 11 g (126 mmol) of morpholine was added and the reaction mixture was stirred at room temperature overnight (18 h). 100 mL of saturated sodium bicarbonate solution was added. The aqueous layer was extracted with CH₂Cl₂ (2 x 50 mL). The combined organic layers were dried (MgSO₄) and concentrated to give 24.3 g (97%) of desired product. ¹H NMR (CDCL₃): δ 9.15 (s, 1H), 8.25 (d, J = 8 Hz, 1H), 7.52 (d, J = 8 Hz, 1H), 4.40 (q, J = 7 Hz, 2H), 3.73 (m, 6H), 2.51 (t, J = 4 Hz, 4H), 1.40 (t, J = 7 Hz, 3H).

### Example 3

**Diester (2).** To a mixture of ester **1** (10.0 g, 40 mmol), diethyl succinate (7.0 g, 40 mmol) and sodium hydride (60% dispersion in mineral oil, 3.20 g, 80 mmol) in dry THF (200 ml) was added DMPU (20 ml) and methanol (0.10 ml) and the mixture was refluxed for 2.5 h. Additional diethyl succinate (10.50 g, 60 mmol) and sodium hydride (4.80 g, 120 mmol) were added in five equal portions to the refluxing reaction mixture at 0.75 h intervals. Refluxing was continued for additional 1.5 h. The cooled reaction mixture was poured into a stirring mixture of 2N HCl (200 ml) and ethyl acetate (200 ml). The aqueous phase was separated, the pH was adjusted to 8.5 with saturated sodium bicarbonate and it was extracted with ethyl acetate. The organic layer was washed with water and dried over anhydrous sodium sulfate. Evaporation of ethyl acetate gave almost pure 2 as yellowish brown oil (10.65 g, 70.4%). ¹H NMR (CDCl₃) δ 1.16 (t, *J* = 7.2 Hz, 3H), 1.22 (t, J= 7.2 Hz, 3H), 2.51-2.53 (m, 4H), 3.02-3.20 (m, 2H), 3.72-3.75 (m, 6H), 4.10-4.15 (m, 4H), 4.78-4.80 (m, 1H), 7.58 (d, *J* = 8.0 Hz, 1H), 8.27 (d, *J =* 6.0 Hz, 1H) and 9.18 (d, *J* = 2.1 Hz, 1H).

### Example 4

**Keto ester (3).** Concentrated sulfuric acid (10 ml) was added carefully to the solution of the diester **2** (11.55 g, 30.5 mmol) in acetic acid (60 ml). The mixture was stirred at 100 °C for 6.5 h. After removing 30-40 ml of acetic acid under reduced pressure, ethanol (125 ml) was added to the residue and the reaction mixture was refluxed for 3.5 h. It was concentrated on a rotary evaporator followed by quenching with water. The mixture was extracted with ethyl acetate. The aqueous layer was separated, treated with saturated NaHCO₃ and extracted with methylene chloride. After drying over anhydrous sodium sulfate, the solvent was evaporated to give 3 as red viscous oil in practically pure state (8.35 g, 89%). Analytically pure sample was obtained from a silica gel column using ethyl acetate/hexane (1:1) as solvent for elution. ¹H NMR (CDCl₃) δ 1.27 (t, *J* = 7.2 Hz, 3H), 2.50-2.53 (m, 4H), 2.76-2.79 (m, 2H), 3.28-3.31 (m, 2H), 3.72-3.75 (m., 6H), 4.15 (q, *J* = 7.2 Hz, 2H), 7.56 (d, *J* = 8.0 Hz, 1H), 8.21 (d, *J* = 6.0 Hz, 1H) and 9.13 (d, *J* = 2.0 Hz, 1H).

### Example 5

**Lactone (4).** Phenylmagnesium bromide (1M/THF, 37.1 mmol, 37.1 ml) was added slowly to a stirring solution of the keto ester 3 (8.11 g, 26.5 mmol) in dry THF at -5°C so that the reaction temperature stayed below 0°C. The reaction mixture was stirred at this temperature for additional 0.5 h. After quenching with 10% ammonium chloride solution, it was extracted with ethyl acetate, dried over anhydrous sodium sulfate and evaporated to give crude **4.** An analytically pure sample was obtained from a silica gel column using ethyl acetate as solvent. ¹H NMR (CDCl₃) δ 2.47-2.49 (m, 4H), 2.58-2.62 (m, 2H), 2.92 (m, 2H), 3.62 (s, 2H), 3.70-3.72 (m, 4H), 7.29-7.42 (m, 6H), 7.69-7.71 (m, 1H) and 8.62 (d, J = 2.0 Hz, 1H).

### Example 6

**Acid (5).** To the solution of crude lactone **4** (from above) in reagent alcohol (100 ml) was added ammonium formate (5.0 g) and 10% Pd/C (0.66 g) and the reaction mixture was refluxed for 2.5 h. The catalyst was filtered and the filtrate was concentrated. A saturated solution of NaHCO₃ was added until the pH was 8.5. It was extracted with ethyl acetate to remove non-acidic impurities. The pH of the aqueous phase was then lowered to 6.5-7 with 2N HCl and it was extracted with CH₂Cl₂, dried over anhydrous sodium sulfate and concentrated to give **5** as light brown viscous oil (3.7 g, 41% over two steps). ¹H NMR (CDCl₃) δ 2.27-2.42 (m, 4H), 2.54-2.56 (m, 4H), 3.65 (s, 2H), 3.71-3.3.73 (m, 4H), 4.09-4.13 (m, 1H), 7.22-7.31 (m, 6H), 7.49-7.51 (m, 1H) and 8.51 (s, 1H).

### Example 7

**Tetralone (6).** A mixture of acid **5** (3.6 g, 10.6 mmol) and polyphosphoric acid (85 g.) was stirred at 110°C for 1.5 h. After cooling, the reaction mixture was quenched with cold water and treated with 2N NaOH to bring the pH up to ∼5. It was extracted with CH₂Cl₂, dried over anhydrous sodium sulfate and evaporated to give almost pure ketone **6** as brown viscous oil (3.1 g, 90%). An analytically pure sample was obtained from preparative TLC using ethyl acetate as a solvent. ¹H NMR (CDCl₃) δ 2.23-2.35 (m, 1H), 2.45-2.78 (m, 7H), 3.68-3.77 (m, 6H), 4.32-4.36 (m, 1H), 6.94 (d, *J* = 6.4 Hz, 1H), 7.34-7.7.47 (m, 4H), 8.13 (d, *J =* 6.4 Hz, 1H) and 8.45 (d, *J* = 2.0 Hz, 1H).

### Example 8

**Oxime (7).** A solution of NaOH (1N, 17.6 ml, 17.6mmol) was added to a stirring solution of hydroxylamine hydrochloride (1.19 g, 17.1 mmol) in water (10 ml) at 0 °C over five minutes followed by addition of the solution of ketone **6** (3.06 g, 9.5 mmol) in reagent alcohol (20 ml). After stirring the reaction mixture at room temperature for 18 h, it was diluted with water and extracted with methylene chloride. The organic layer was dried over anhydrous sodium sulfate and evaporated to give crude product. It was purified by silica gel chromatography using ethyl acetate as the solvent to give 7 as a colorless oil. It solidified on standing (2.00 g, 62%). ¹H NMR (CDCl₃) δ 2.02-2.12 (m, 1H), 2.16-2.26 (m, 1H), 2.50-2.60 (m, 4H), 2.70-2.90 (m, 2H), 3.7 (s, 2H), 3.72-3.78 (m, 4H), 4.15-4.20 (m, 4H), 6.90-6.95 (m, 1H), 7.20-7.35 (m, 3H), 7.98-8.02 (m, 1H), 8.4 (s, 1H) and 9.05 (bs, 1H).

### Example 9

**Amide (8).** A solution of oxime **7** (1.42 g, 4.2 mmol) in acetic anhydride (15 ml) and acetic acid (1ml, 16.8mmol) was stirred at room temperature for 0.5 h. Iron powder (0.63 g, 10 mgatom) was added and the mixture was stirred at 55 °C for 5 h. The reaction mixture was cooled, ethyl acetate was added and the resulting mixture was filtered. The filtrate was evaporated to dryness. Water was added, the pH was adjusted to ∼8 with 2N NaOH and again extracted with ethyl acetate. It was dried over anhydrous sodium sulfate and concentrated. The crude product was purified by silica gel chromatography eluting with 2.5% MeOH/CH₂Cl₂ to give **8** as yellow viscous oil (1.08 g, 71%). ¹H NMR (CDCl₃) 2.18 (s, 3H), 2.50-2.51 (m, 4H), 2.63-2.66 (m, 2H), 3.62-3.73 (m, 6H), 6.30-6.31 (m, 1H), 6.87-6.97 (m, 2H), 7.14-7.33 (m, 5H), 7.51-7.53 (m, 1H) and 8.41 (bs, 1H).

### Example 10

**N-Acetyl naphthalene (9).** A solution of amide **8** (0.36 g, 0.99 mmol) in methylene chloride (5 ml) was added fairly rapidly to a suspension of DDQ (0.34 g, 1.5 mmol) in methylene chloride (15 ml) at room temperature. After stirring the black reaction mixture for 0.25 h., it was quenched with NaOH (2N, 7 ml). The organic phase was separated, dried and evaporated to give yellow residue. It was passed through a plug of silica gel to give pure **9** (0.21 g, 59%). ¹H NMR (CDCl₃) δ 2.37 (s, 3H), 2.59-2.62 (m, 4H), 3.70-3.79 (m, 6H), 7.35-7.55 (m, 5H), 7.75-7.96 (m, 4H) and 8.66 (bs, 1H).

### Example 11

**Naphthyl amine (10).** An aqueous solution of NaOH (3N, 6 ml) was added to a stirring solution of 9 (0.195 g, 0.54 mmol) in reagent alcohol (4 ml) and the mixture was refluxed for 5 h. The reaction mixture was cooled, diluted with water and extracted with methylene chloride. The organic layer was dried over anhydrous sodium sulfate and evaporated to give **10** as yellow foam (014 g, 80%). ¹H NMR (CDCl₃) δ 2.59-2.61 (m, 4H), 3.75- 3.80 (m, 6H), 4.28 (bs, 2H), 6.85 (d, *J* = 7.60 Hz, 1H), 7.23-7.26 (m, 1H), 7.44-7.51 (m, 3H), 7.75-7.77 (m, 1H), 7.82 (d, *J =* 8.0 Hz, 1H), 7.89 (d, *J =* 8.0 Hz, 1H) and 8.67 (d, *J* = 2.0,1H).

## Claims

1. A process of making a compound of the formula(I): 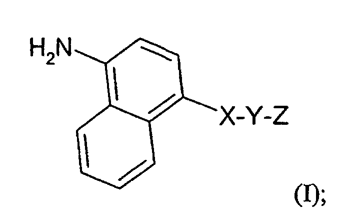 wherein:
the naphthyl ring is further optionally substituted by one or more R₁ or R₂;
X is chosen from
a C₅₋₈ cycloalkyl and cycloalkenyl optionally substituted with one to two oxo groups or one to three C₁₋₄ alkyl, C₁₋₄ alkoxy or C₁₋₄ alkylamino chains each being branched or unbranched;
aryl, furanyl, thienyl, pyrrolyl, pyrazolyl, imidazolyl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl and pyrazinyl; each being optionally independently substituted with one to three C₁₋₄ alkyl, C₁₋₄alkoxy, hydroxy, nitro, nitrile, amino, mono- or di-(C₁₋₃ alkyl)amino, mono- or di-(C₁₋₃ alkylamino)carbonyl, NH₂C(O), C₁₋₆ alkyl-S(O)ₘ or halogen;
Y is chosen from
a bond and a C₁₋₄ saturated or unsaturated branched or unbranched carbon chain optionally partially or fully halogenated, wherein one or more methylene groups are optionally replaced by O, N, or S(O)ₘ and wherein Y is optionally independently substituted with one to two oxo groups, phenyl or one or more C₁₋₄ alkyl optionally substituted by one or more halogen atoms;
Z is chosen from
phenyl, pyridinyl, pyrimidinyl, pyridazinyl, pyrazinyl, imidazolyl, pyrazolyl, triazolyl, tetrazolyl, furanyl, thienyl, pyranyl, each being optionally substituted with one to three halogen, C₁₋₆ alkyl, C₁₋₆ alkoxy, hydroxy, amino, mono- or di-(C₁₋₃ alkyl)amino, C₁₋₆ alkyl-S(O)ₘ, CN, CONH₂, COOH or phenylamino wherein the phenyl ring is optionally substituted with one to two halogen, C₁₋₆ alkyl or C₁₋₆ alkoxy;
tetrahydropyranyl, tetrahydrofuranyl, 1,3-dioxolanonyl, 1,3-dioxanonyl, 1,4-dioxanyl, morpholinyl, thiomorpholinyl, thiomorpholinyl sulfoxidyl, thiomorpholinyl sulfonyl, piperidinyl, piperidinonyl, piperazinyl, tetrahydropyrimidonyl, cyclohexanonyl, cyclohexanolyl, pentamethylene sulfidyl, pentamethylene sulfoxidyl, pentamethylene sulfonyl, tetramethylene sulfide, tetramethylene sulfoxidyl or tetramethylene sulfonyl each being optionally substituted with one to three nitrile, C₁₋₆ alkyl, C₁₋₆ alkoxy, hydroxy, amino, mono- or di-(C₁₋₃ alkyl)amino-C₁₋₃ alkyl, CONH₂, phenylamino-C₁₋₃ alkyl or C₁₋₃ alkoxy-C₁₋₃ alkyl;
halogen, C₁₋₄ alkyl, nitrile, amino, hydroxy, C₁₋₆ alkoxy, NH₂C(O), mono- or di(C₁₋₃alkyl) aminocarbonyl, mono- or di(C₁₋₃alkyl)amino, secondary or tertiary amine wherein the amino nitrogen is covalently bonded to C₁₋₃ alkyl or C₁₋₅ alkoxyalkyl, pyridinyl-C₁₋₃ alkyl, imidazolyl-C₁₋₃ alkyl, tetrahydrofuranyl-C₁₋₃ alkyl, nitrile-C₁₋₃ alkyl, carboxamide-C₁₋₃ alkyl, phenyl, wherein the phenyl ring is optionally substituted with one to two halogen, C₁₋₆ alkoxy, hydroxy or mono- or di-(C₁₋₃ alkyl)amino, C₁₋₆ alkyl-S(O)ₘ, or phenyl-S(O)ₘ, wherein the phenyl ring is optionally substituted with one to two halogen, C₁₋₆ alkoxy, hydroxy, halogen or mono- or di-(C₁₋₃ alkyl)amino;
C₁₋₆ alkyl-S(O)ₘ, and phenyl-S(O)ₘ, wherein the phenyl ring is optionally substituted with one to two halogen, C₁₋₆ alkoxy, hydroxy or mono- or di-(C₁₋₃ alkyl)amino;
R₁ and R₂ are independently chosen from:
a C₁₋₆ branched or unbranched alkyl optionally partially or fully halogenated, C₁₋₄ branched or unbranched alkoxy, each being optionally partially or fully halogenated, halogen, C₁₋₃ alkyl-S(O)ₘ optionally partially or fully halogenated and phenylsulfonyl;
m is 0, 1 or 2;
said process comprising:
i) reacting a Z-Y-X-COO-Rₓ ester (II) with a di-ester (III), wherein Rₓ in both (II) and (III) is independently C₁₋₅alkyl or aryl, in a suitable solvent and a suitable base and optionally in the presence of an additive at a temperature of 0 to 200°C for a reaction time of 5 min to 24 h, and isolating the compound intermediate (IV): 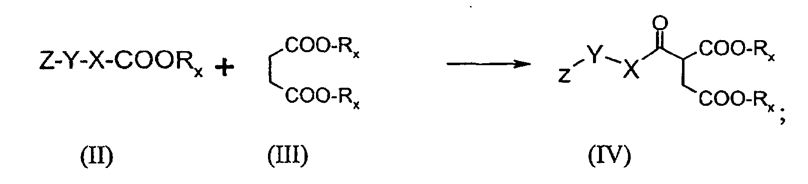
ii) hydrolysing the product of step i) with acidic or basic hydrolysis, and decarboxylating under suitable acid conditions at a temperature of about 50 to 200 °C and for 5 min to 24 hours; followed by esterifying under appropriate conditions with a C₁₋₅alcohol; subsequent phenyl nucleophilic addition wherein the phenyl is optionally independently substituted by R₁ and R₂; cleaving by reductive cleavage under appropriate conditions to form a carboxylic acid compound, and treating with a strong mineral acid optionally in a suitable solvent at RT to 200°C, to form a product intermediate of the fornmla(V), and isolating the product: 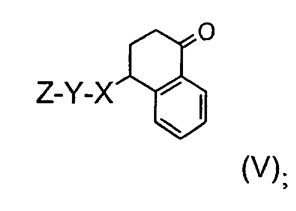
iii) reacting the product from step ii) with HNR_{y}R_{z} or it's respective salt thereof, to form an enamine or imine compound of the formuIa(VI) under suitable conditions at RT for 1 to 24 h: 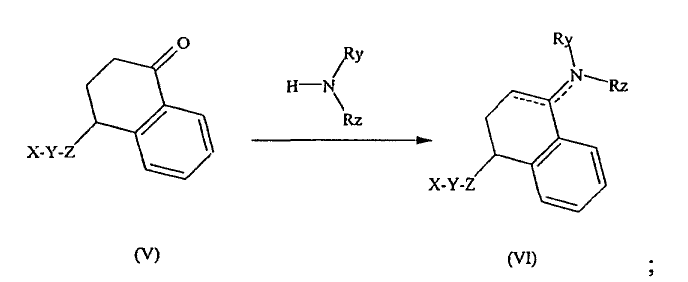 wherein R_{y} is C₁₋₅alkyl or hydrogen, R_{z} is C₁₋₅alkyl, hydrogen or OH with the proviso that when formula (VI) is an enamine tautomer then R_{y} and R_{z} are both C₁₋₅alkyl, or when formula (VI) is an imine tautomer then R_{z} is OH, C₁₋₅alkyl or hydrogen and R_{y} is not present: 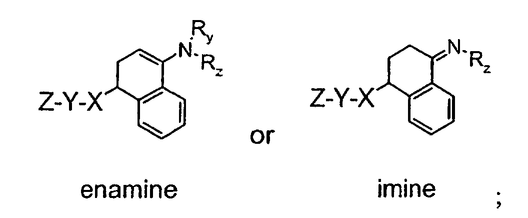
iv) where the product of step iii) is an imine, in a one pot reaction acylating and reducing the product (VI); then treating the product of such reaction under oxidizing conditions capable of forming the naphthalene ring in the formula(I), followed by deprotecting under suitable conditions;
or
where the product of step iii) is an enamine, oxidizing the enamine under suitable oxidizing conditions to form the naphthalene ring, then deprotecting the amino nitrogen to form the fomiula (I):

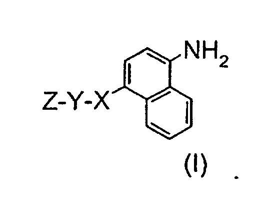

2. The process according to claim 1 wherein:
X is chosen from
a C₅₋₈ cycloalkyl and cycloalkenyl optionally substituted with one to two oxo groups or one to three C₁₋₄ alkyl, C₁₋₄ alkoxy or C₁₋₄ alkylamino chains each being branched or unbranched;
aryl, pyridinyl, pyrimidinyl, pyridinonyl, dihydropyridinonyl, maleimidyl, dihydromaleimidyl, piperdinyl, benzimidazole, 3H-imidazo[4,5-b]pyridine, piperazinyl, pyridazinyl and pyrazinyl; each being optionally independently substituted with one to three C₁₋₄ alkyl, nitro, nitrile, mono- or di-(C₁₋₃ alkyl)amino, mono- or di-(C₁₋₃ alkylamino)carbonyl, NH₂C(O), C₁₋₆ alkyl-S(O)ₘ or halogen;
Y is chosen from
a bond and
a C₁₋₄ saturated or unsaturated carbon chain wherein one of the carbon atoms is optionally replaced by O, N, or S(O)ₘ and wherein Y is optionally independently substituted with one to two oxo groups, phenyl or one or more C₁₋₄ alkyl optionally substituted by one or more halogen atoms;
Z is chosen from
phenyl, pyridinyl, pyrimidinyl, pyridazinyl, pyrazinyl, imidazolyl, furanyl, thienyl, dihydrothiazolyl, dihydrothiazolyl sulfoxidyl, pyranyl, pyrrolidinyl which are optionally substituted with one to three nitrile, C₁₋₃ alkyl, C₁₋₃ alkoxy, amino, hydroxy, mono- or di- (C₁₋₃ alkyl)amino or CONH₂;
tetrahydropyranyl, tetrahydrofuranyl, 1,3-dioxolanonyl, 1,3-dioxanonyl, 1,4-dioxanyl, morpholinyl, thiomorpholinyl, thiomorpholinyl sulfoxidyl, piperidinyl, piperidinonyl, piperazinyl, tetrahydropyrimidonyl, pentamethylene sulfidyl, pentamethylene sulfoxidyl, pentamethylene sulfonyl, tetramethylene sulfidyl, tetramethylene sulfoxidyl or tetramethylene sulfonyl which are optionally substituted with one to three nitrile, C₁₋₃ alkyl, C₁₋₃ alkoxy, hydroxy, amino, mono- or di-(C₁₋₃ alkyl)amino or CONH₂; nitrile, C₁₋₆ alkyl-S(O)ₘ, halogen, hydroxy, C₁₋₄ alkoxy, amino, mono- or di-(C₁₋₆ alkyl)amino, mono- or di-(C₁₋₃ alkyl)aminocarbonyl and NH₂C(O).

3. The process according to claim 2 wherein:
X is
aryl, pyridinyl, pyrimidinyl, benzimidazole, 3H-imidazo[4,5-b]pyridine, piperazinyl, pyridazinyl and pyrazinyl; each being optionally independently substituted with one to three C₁₋₄ alkyl, nitro, nitrile, mono- or di-(C₁₋₃ alkyl)amino, mono- or di-(C₁₋₃ alkylamino)carbonyl, NH₂C(O), C₁₋₆ alkyl-S(O)ₘ or halogen;
Y is chosen from
a bond and
a C₁₋₄ saturated carbon chain wherein one of the carbon atoms is optionally replaced by O, N or S and wherein Y is optionally independently substituted with an oxo group;
Z is chosen from
phenyl, pyridinyl, pyrimidinyl, pyridazinyl, pyrazinyl, imidazolyl, dihydrothiazolyl, dihydrothiazolyl sulfoxide, pyranyl and pyrrolidinyl which are optionally substituted with one to two C₁₋₂ alkyl or C₁₋₂ alkoxy;
tetrahydropyranyl, morpholinyl, thiomorpholinyl, thiomorpholinyl sulfoxidyl, piperidinyl, piperidinonyl, piperazinyl and tetrahydropyrimidonyl which are optionally substituted with one to two C₁₋₂ alkyl or C₁₋₂ alkoxy; and C₁₋₃ alkoxy.

4. The process according to claim 3 wherein
X is chosen from
pyridinyl and pyrimidinyl, each being optionally independently substituted with one to three C₁₋₄ alkyl, nitro, nitrile, mono- or di-(C₁₋₃ alkyl)amino, mono- or di-(C₁₋₃ alkylamino)carbonyl, NH₂C(O), C₁₋₆ alkyl-S(O)ₘ or halogen;
Y is chosen from
a bond, -CH₂-, -CH₂CH₂-, -C(O)-, -O-, -S-, -NH-CH₂CH₂CH₂-, -N(CH₃)- and -NH-.

5. The process according to claim wherein:
Y is chosen from
-CH₂-, -NH-CH₂CH₂CH₂- and -NH- and
Z is morpholinyl.

6. The process according to claims 1,2,3,4 or 5 wherein:
in step i) :
Rₓ is C₂H₅;
the solvent is aprotic chosen from THF, DME, DMSO, ether, dioxane, CH₂Cl₂, CHCl₃, toluene, pyridine and DMF;
the base is chosen from NaH, NaNH₂, Na t-butoxide, NaOH, pyridine, TEA, Na ethoxide, DBU and BuLi;
the additive is chosen from DMPU and HMPA;
and the reaction time is 8 h.
in step ii):
the hydrolysis is acidic;
the decarboxylation acidic conditions are conc. H₂SO₄ in HOAc, the temperature is 100°C and the time is 7 h;
the esterification is with CH₃CH₂OH;
the phenyl nucleophilic addition is via a phenyl Grignard reagent PhMgBr;
the reductive cleavage is with HCOONH₄ / Pd/C / EtOH;
the added mineral acid is PPA and at a temperature of 110°C;
in step iii):
forming an oxime under oxime formation conditions by adding HNR_{y}R_{z} wherein said HNRyR_{z} is the amine salt NH₂OH.HCl, in EtOH with NaOH for 18 h;
in step iv):
the product of step iii) is acetylated with acetic anhydride and acetic acid then reducing compound (VI) with Fe at 55°C for 5 hours;
the oxidizing conditions are treating the product with DDQ in a nonpolar solvent, at 0 to 50 °C, at 0.5 to 10 h.

7. The process according to claim 6 wherein:
in step i) :
the solvent is THF;
the temperature is 60-70 °C;
the base is chosen from NaH and Na t-butoxide;
the additive is DMPU;
and
in step iv):
for the oxidizing step the the nonpolar solvent is methylene chloride, the temperature is about RT and the time is 5 h.

## Patentansprüche

1. Verfahren zur Herstellung einer Verbindung der Formel(I): 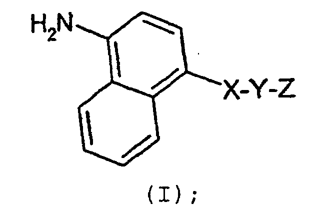 wobei:
der Naphthylring gegebenenfalls zudem mit einem oder mehreren R₁ oder R₂ substituiert ist;
X ausgewählt ist aus
einem C₅₋₈-Cycloalkyl und Cycloalkenyl, das gegebenenfalls substituiert ist mit einer bis zwei Oxo-Gruppen oder einer bis drei C₁₋₄-Alkyl-, C₁₋₄-Alkoxy- oder C₁₋₄-Alkylaminoketten, wovon jede verzweigt oder unverzweigt ist;
Aryl, Furanyl, Thienyl, Pyrrolyl, Pyrazolyl, Imidazolyl, Pyridinyl, Pyrimidinyl, Pyridinonyl, Dihydropyridinonyl, Maleimidyl, Dihydromaleimidyl, Piperdinyl, Benzimidazol, 3H-Imidazo[4,5-b]pyridin, Piperazinyl, Pyridazinyl und Pyrazinyl; wobei jedes gegebenenfalls unabhängig mit einem bis drei C₁₋₄-Alkyl, C₁₋₄-Alkoxy, Hydroxy, Nitro, Nitril, Amino, Mono- oder Di-(C₁₋₃-Alkyl)amino, Mono- oder Di-(C₁₋₃-Alkylamino)carbonyl, NH₂C(O), C₁₋₆-Alkyl-S(O)ₘ oder Halogen substituiert ist;
Y ausgewählt ist aus
einer Bindung und einer gesättigten oder ungesättigten, verzweigten oder unverzweigten, gegebenenfalls partiell oder vollständig halogenierten C₁₋₄-Kohlenstoffkette, wobei eine oder mehrere Methylengruppen gegebenenfalls mit O, N oder S(O)ₘ ersetzt sind, und wobei Y gegebenenfalls unabhängig mit einer bis zwei Oxo-Gruppen, Phenyl oder einem oder mehreren, gegebenenfalls mit einem oder mehreren Halogenatomen substituierten C₁₋₄-Alkyl substituiert ist;
Z ausgewählt ist aus
Phenyl, Pyridinyl, Pyrimidinyl, Pyridazinyl, Pyrazinyl, Imidazolyl, Pyrazolyl, Triazolyl, Tetrazolyl, Furanyl, Thienyl, Pyranyl, wobei jedes gegebenenfalls unabhängig substituiert ist mit einem bis drei Halogen, C₁₋₆-Alkyl, C₁₋₆-Alkoxy, Hydroxy, Amino, Mono- oder Di-(C₁₋₃-Alkyl)amino, C₁₋₆-Alkyl-S(O)ₘ, CN, CONH₂, COOH oder Phenylamino, wobei der Phenylring gegebenenfalls mit einem bis zwei Halogen, C₁₋₆-Alkyl oder C₁₋₆-Alkoxy substituiert ist;
Tetrahydropyranyl, Tetrahydrofuranyl, 1,3-Dioxolanonyl, 1,3-Dioxanonyl, 1,4-Dioxanyl, Morpholinyl, Thiomorpholinyl, Thiomorpholinylsulfoxidyl, Thiomorpholinylsulfonyl, Piperidinyl, Piperidinonyl, Piperazinyl, Tetrahydropyrimidonyl, Cyclohexanonyl, Cyclohexanolyl, Pentamethylensulfidyl, Pentamethylensulfoxidyl, Pentamethylensulfonyl, Tetramethylensulfid, Tetramethylensulfoxidyl oder Tetramethylensulfonyl, wobei jedes gegebenenfalls unabhängig mit einem bis drei Nitrilen, C₁₋₆-Alkyl, C₁₋₆-Alkoxy, Hydroxy, Amino, Mono- oder Di-(C₁₋₃-Alkyl)amino-C₁₋₃-Alkyl, CONH₂, Phenylamino-C₁₋₃-alkyl oder C₁₋₃-Alkoxy-C₁₋₃-alkyl substituiert ist;
Halogen, C₁₋₄-Alkyl, Nitril, Amino, Hydroxy, C₁₋₆-Alkoxy, NH₂C(O), Mono- oder Di-(C₁₋₃-Alkyl)aminocarbonyl, Mono- oder Di-(C₁₋₃-Alkyl)amino, sekundäres oder tertiäres Amin, wobei der Amin-Stickstoff kovalent an C₁₋₃-Alkyl oder C₁₋₅-Alkoxyalkyl gebunden ist, Pyridinyl-C₁₋₃-Alkyl, Imidazolyl-C₁₋₃-Alkyl, Tetrahydrofuranyl-C₁₋₃-Alkyl, Nitril-C₁₋₃-Alkyl, Carboxamid-C₁₋₃-Alkyl, Phenyl, wobei der Phenylring gegebenenfalls mit einem bis zwei Halogenen, C₁₋₆-Alkoxy, Hydroxy oder Mono- oder Di-(C₁₋₃-Alkyl)amino substituiert ist, C₁₋₆-Alkyl-S(O)ₘ oder Phenyl-S(O)ₘ, wobei der Phenylring gegebenenfalls substituiert ist mit einem bis zwei Halogenen, C₁₋₆-Alkoxy, Hydroxy, Halogen oder Mono- oder Di- (C₁₋₃-Alkyl) amino;
C₁₋₆-Alkyl-S(O)ₘ und Phenyl-S(O)ₘ, wobei der Phenylring gegebenenfalls mit einem bis zwei Halogenen, C₁₋₆-Alkoxy, Hydroxy oder Mono- oder Di-(C₁₋₃-Alkyl)amino substituiert ist;
R₁ und R₂ unabhängig ausgewählt sind aus:
einem verzweigten oder unverzweigten, gegebenenfalls partiell oder vollständig halogenierten C₁₋₆-Alkyl, einem verzweigten oder unverzweigten, gegebenenfalls partiell oder vollständig halogenierten C₁₋₄-Alkoxy, einem Halogen, einem gegebenenfalls partiell oder vollständig halogenierten C₁₋₃-Alkyl-S(O)ₘ und Phenylsulfonyl;
m 0, 1 oder 2 ist;
und wobei besagtes Verfahren umfasst:
i) Umsetzen eines Z-Y-X-COOR-Rₓ-Esters (II) mit einem Diester (III), wobei Rₓ sowohl in (II) als auch in (III) unabhängig C₁₋₅-Alkyl oder Aryl ist, in einem geeigneten Lösungsmittel und einer geeigneten Base und gegebenenfalls in der Anwesenheit eines Additivs bei einer Temperatur von 0 bis 200°C während einer Reaktionszeit von 5 Min. bis 24 Std. und Isolieren des Zwischenproduktes (IV): 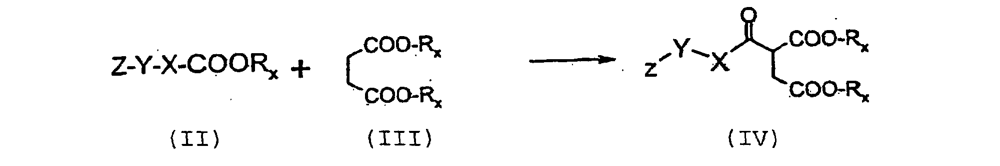
ii) Hydrolysieren des Produktes von Schritt i) mit saurer oder basischer Hydrolyse und Decarboxylierung unter geeigneten sauren Bedingungen bei einer Temperatur von ungefähr 50 bis 200°C während 5 Min. bis 24 Stunden; gefolgt von Veresterung unter geeigneten Bedingungen mit einem C₁₋₅-Alkohol; anschliessende nukleophile Addition von Phenyl, wobei das Phenyl gegebenenfalls unabhängig mit R₁ und R₂ substituiert ist; Spaltung durch reduktives Spalten unter geeigneten Bedingungen, woraus sich eine Carbonsäure-Verbindung bildet, und Behandlung mit einer starken Mineralsäure, gegebenenfalls in einem geeigneten Lösungsmittel, bei RT bis 200°C, woraus sich ein Zwischenprodukt der Formel (V) bildet, und Isolieren des Produktes: 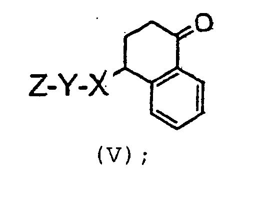
iii) Umsetzen des Produktes von Schritt ii) mit HNR_{y}R_{z} oder eines entsprechenden Salzes davon, woraus sich unter geeigneten Bedingungen bei RT während 1 bis 24 Std. eine Enamin- oder Imin-Verbindung der Formel (VI) bildet: 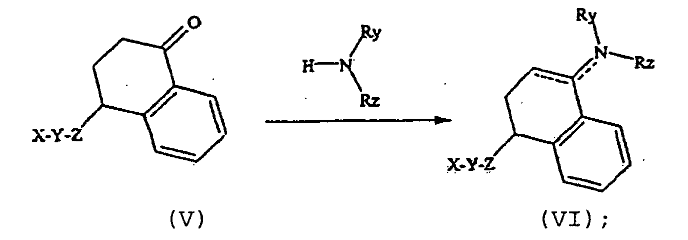 wobei R_{y} C₁₋₅-Alkyl oder Wasserstoff ist, R_{z} C₁₋₅-Alkyl, Wasserstoff oder OH ist mit der Massgabe, dass wenn Formel (VI) ein Enamin-Tautomer ist, dann R_{y} und R_{z} beide C₁₋₅-Alkyl sind, oder dass wenn Formel (VI) ein Imin-Tautomer ist, dann R_{z} OH, C₁₋₅-Alkyl oder Wasserstoff und R_{y} nicht vorhanden ist: 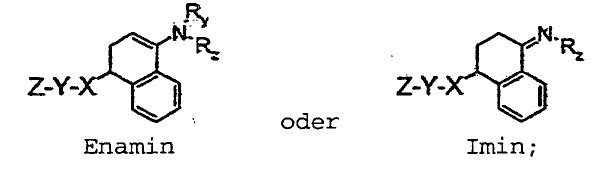
iv) falls das Produkt von Schritt iii) ein Imin ist, Acylieren und Reduzieren des Produktes (VI) in einer Einbehälter-Reaktion; anschliessendes Behandeln des Produktes einer solchen Reaktion unter Oxidationsbedingungen, die zur Bildung eines Naphthalinrings in der Formel (I) befähigt sind, gefolgt von Entschützen unter geeigneten Bedingungen;
falls das Produkt von Schritt iii) ein Enamin ist, Oxidieren des Enamins unter geeigneten Oxidationsbedingungen zur Bildung des Naphthalinrings, und anschliessendes Entschützen des Amino-Stickstoffes unter Bildung der Formel (I):

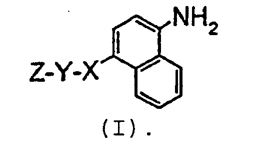

2. Verfahren nach Anspruch 1, wobei:
X ausgewählt ist aus
einem C₅₋₈-Cycloalkyl und Cycloalkenyl, das gegebenenfalls substituiert ist mit einer bis zwei Oxo-Gruppen oder einer bis drei C₁₋₄-Alkyl-, C₁₋₄-Alkoxy- oder C₁₋₄-Alkylaminoketten, wobei jede verzweigt oder unverzweigt ist;
Aryl, Pyridinyl, Pyrimidinyl, Pyridinonyl, Dihydropyridinonyl, Maleimidyl, Dihydromaleimidyl, Piperdinyl, Benzimidazol, 3H-Imidazo[4,5-b]pyridin, Piperazinyl, Pyridazinyl und Pyrazinyl; wobei jedes gegebenenfalls unabhängig mit einem bis drei C₁₋₄-Alkyl, Nitro, Nitril, Mono- oder Di-(C₁₋₃-Alkyl)amino, Mono- oder Di-(C₁₋₃-Alkylamino)carbonyl, NH₂C(O), C₁₋₆-Alkyl-S(O)ₘ oder Halogen substituiert ist;
Y ausgewählt ist aus
einer Bindung und
einer gesättigten oder ungesättigten C₁₋₄-Kohlenstoffkette, wobei eines der Kohlenstoffatome gegebenenfalls mit O, N oder S(O)ₘ ersetzt ist, und wobei Y gegebenenfalls unabhängig mit einer bis zwei Oxo-Gruppen, Phenyl oder einem oder mehreren, gegebenenfalls mit einem oder mehreren Halogenatomen substituierten C₁₋₄-Alkyl substituiert ist;
Z ausgewählt ist aus
Phenyl, Pyridinyl, Pyrimidinyl, Pyridazinyl, Pyrazinyl, Imidazolyl, Furanyl, Thienyl, Dihydrothiazolyl, Dihydrothiazolylsulfoxidyl, Pyranyl, Pyrrolidinyl, welche gegebenenfalls substituiert sind mit einem bis drei Nitrilen, C₁₋₃-Alkyl, C₁₋₃-Alkoxy, Amino, Hydroxy, Mono- oder Di-(C₁₋₃-Alkyl)amino oder CONH₂;
Tetrahydropyranyl, Tetrahydrofuranyl, 1,3-Dioxolanonyl, 1,3-Dioxanonyl, 1,4-Dioxanyl, Morpholinyl, Thiomorpholinyl, Thiomorpholinylsulfoxidyl, Piperidinyl, Piperidinonyl, Piperazinyl, Tetrahydropyrimidonyl, Pentamethylensulfidyl, Pentamethylensulfoxidyl, Pentamethylensulfonyl, Tetramethylensulfidyl, Tetramethylensulfoxidyl oder Tetramethylensulfonyl, welche gegebenenfalls substituiert sind mit einem bis drei Nitril, C₁₋₃-Alkyl, C₁₋₃-Alkoxy, Hydroxy, Amino, Mono- oder Di-(C₁₋₃-Alkyl)amino oder CONH₂; Nitril, C₁₋₆-Alkyl-S(O)ₘ, Halogen, Hydroxy, C₁₋₄-Alkoxy, Amino, Mono- oder Di-(C₁₋₆-Alkyl)amino, Mono- oder Di-(C₁₋₃-Alkyl)aminocarbonyl und NH₂C(O).

3. Verfahren nach Anspruch 2, wobei:
X
Aryl, Pyridinyl, Pyrimidinyl, Benzimidazol, 3H-Imidazo[4,5-b]pyridin, Piperazinyl, Pyridazinyl und Pyrazinyl ist, wobei jedes gegebenenfalls unabhängig substituiert ist mit einem bis drei C₁₋₄-Alkyl, Nitro, Nitril, Mono- oder Di-(C₁₋₃-Alkyl)amino, Mono- oder Di-(C₁₋₃-Alkylamino)carbonyl, NH₂C(O), C₁₋₆-Alkyl-S(O)ₘ oder Halogen;
Y ausgewählt ist aus
einer Bindung und
einer gesättigten C₁₋₄-Kohlenstoffkette, wobei eines der Kohlenstoffatome gegebenenfalls mit O, N oder S ersetzt ist, und wobei Y gegebenenfalls unabhängig mit einer Oxo-Gruppe substituiert ist;
Z ausgewählt ist aus
Phenyl, Pyridinyl, Pyrimidinyl, Pyridazinyl, Pyrazinyl, Imidazolyl, Dihydrothiazolyl, Dihydrothiazolylsulfoxid, Pyranyl und Pyrrolidinyl, welche gegebenenfalls unabhängig mit einem bis zwei C₁₋₂-Alkyl oder C₁₋₂-Alkoxy substituiert sind;
Tetrahydropyranyl, Morpholinyl, Thiomorpholinyl, Thiomorpholinylsulfoxidyl, Piperidinyl, Piperidinonyl, Piperazinyl und Tetrahydropyrimidonyl, welche gegebenenfalls substituiert sind mit einem bis zwei C₁₋₂-Alkyl oder C₁₋₂-Alkoxy; und
C₁₋₃-Alkoxy.

4. Verfahren nach Anspruch 3, wobei
X ausgewählt ist aus
Pyridinyl und Pyrimidinyl, wobei jedes gegebenenfalls unabhängig mit einem bis drei C₁₋₄-Alkyl, Nitro, Nitril, Mono- oder Di-(C₁₋₃-Alkyl)amino, Mono- oder Di-(C₁₋₃-Alkylamino)carbonyl, NH₂C(O), C₁₋₆-Alkyl-S(O)ₘ oder Halogen substituiert ist;
Y ausgewählt ist aus
einer Bindung, -CH₂-, -CH₂CH₂, -C(O)-, -O-, -S-, -NH-CH₂CH₂CH₂-, -N(CH₃)- und -NH-.

5. Verfahren nach Anspruch 4, wobei
Y ausgewählt ist aus
-CH₂-, -NH-CH₂CH₂CH₂- und -NH- und
Z Morpholinyl ist.

6. Verfahren nach Anspruch 1, 2, 3, 4 oder 5, wobei
in Schritt i):
Rₓ C₂H₅ ist;
das Lösungsmittel aprotisch und aus THF, DME, DMSO, Ether, Dioxan, CH₂Cl₂, CHCl₃, Toluol, Pyridin und DMF ausgewählt ist;
die Base aus NaH, NaNH₂, Na-t-Butoxid, NaOH, Pyridin, TEA, Na-ethoxid, DBU und BuLi ausgewählt ist;
das Additiv aus DMPU und HMPA ausgewählt ist;
und die Reaktionszeit 8 Std. ist.
in Schritt ii):
die Hydrolyse sauer ist;
die sauren Decarboxylierungsbedingungen konz. H₂SO₄ in HOAc sind, die Temperatur 100°C ist und die Zeit 7 Std. ist;
die Veresterung mit CH₃CH₂OH ist;
die nukleophile Phenyladdition über ein Phenyl-Grignard-Reagens PhMgBr ist;
die reduktive Spaltung mit HCOONH₄/Pd/C/EtOH ist;
die zugegebene Mineralsäure PPA ist und bei einer Temperatur von 110°C;
in Schritt iii):
Bildung eines Oxims unter Oxim-Bildungsbedingungen durch Zugabe von HNR_{y}R_{z}, wobei besagtes HNRyR_{z} das Aminsalz NH₂OH·HCl in EtOH mit NaOH während 18 Std. ist;
in Schritt iv):
das Produkt von Schritt iii) mit Essigsäureanhydrid und Essigsäure acetyliert wird, dann die Verbindung (VI) mit Fe bei 55°C während 5 Stunden reduziert wird;
die Oxidationsbedingungen das Behandeln des Produktes mit DDQ in einem nicht-polaren Lösungsmittel bei 0 bis 50°C während 0.5 bis 10 Std. sind.

7. Verfahren nach Anspruch 6, wobei
in Schritt i):
das Lösungsmittel THF ist;
die Temperatur 60-70°C ist;
die Base aus NaH und Na-t-Butoxid ausgewählt ist;
das Additiv DMPU ist;
und
in Schritt iv):
für den Oxidationsschritt das nicht-polare Lösungsmittel Methylenchlorid ist, die Temperatur ungefähr RT ist und die Zeit 5 Std. ist.

## Revendications

1. Procédé de production d'un composé de formule (I) : 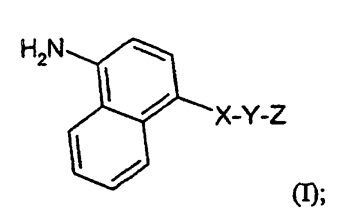 où :
le cycle naphtyle est encore éventuellement substitué par un ou plusieurs R₁ ou R₂ ;
X est choisi parmi
un C₅₋₈ cycloalkyle et cycloalcényle éventuellement substitué avec un ou deux groupes oxo ou une à trois chaînes C₁₋₄ alkyle, C₁₋₄ alcoxy ou C₁₋₄ alkylamino chacune étant ramifiée ou non ramifiée ;
aryle, furanyle, thiényle, pyrrolyle, pyrazolyle, imidazolyle, pyridinyle, pyrimidinyle, pyridinonyle, dihydropyridinonyle, maléimidyle, dihydromaléimidyle, pipéridinyle, benzimidazole, 3H-imidazo[4,5-b]pyridine, pipérazinyle, pyridazinyle et pyrazinyle ; chacun étant éventuellement substitué indépendamment avec un à trois C₁₋₄ alkyle, C₁₋₄ alcoxy, hydroxyle, nitro, nitrile, amino, mono- ou di-(C₁₋₃ alkyl)amino, mono- ou dl-(C₁₋₃ alkylamino)carbonyle, NH₂C(O), C₁₋₆ alkyl-S(O)ₘ ou halogène ;
Y est choisi parmi
une liaison et une chaîne carbonée C₁₋₄ saturée ou insaturée, ramifiée ou non ramifiée éventuellement partiellement ou totalement halogénée, où un ou plusieurs groupes méthylène sont éventuellement remplacés par O, N ou S(O)ₘ et où Y est éventuellement indépendamment substitué avec un ou deux groupes oxo, phényle ou un ou plusieurs C₁₋₄ alkyle éventuellement substitués par un ou plusieurs atomes d'halogène ;
Z est choisi parmi
phényle, pyridinyle, pyrimidinyle, pyridazinyle, pyrazinyle, imidazolyle, pyrazolyle, triazolyle, tétrazolyle, furanyle, thiényle, pyranyle, chacun étant éventuellement substitué avec un à trois halogènes, C₁₋₆ alkyle, C₁₋₆ alcoxy, hydroxyle, amino, mono- ou di-( C₁₋₃ alkyl)amino, C₁₋₆ alkyl-S(O)ₘ, CN, CONH₂, COOH ou phénylamino où le cycle phényle est éventuellement substitué avec un à deux halogènes, C₁₋₆ alkyle ou C₁₋₆ alcoxy ;
tétrahydropyranyle, tétrahydrofuranyle, 1,3-dioxolanonyle, 1,3-dioxanonyle, 1,4-dioxanyle, morpholinyle, thiomorpholinyle, thiomorpholinyl sulfoxydyle, thiomorpholinyl sulfonyle, pipéridinyle, pipéridinonyle, pipérazinyle, tétrahydropyrimidonyle, cyclohexanonyle, cyclohexanolyle, pentaméthylène sulfuryle, pentaméthylène sulfoxydyle, pentaméthylène sulfonyle, tétraméthylène sulfure, tétraméthylène sulfoxydyle ou tétraméthylène sulfonyle chacun étant éventuellement substitué avec un à trois nitriles, C₁₋₆ alkyle, C₁₋₆ alcoxy, hydroxyle, amino, mono- ou di-(C₁₋₃ alkyl)amino-C₁₋₃ alkyle, CONH₂, phénylamino-C₁₋₃ alkyle ou C₁₋₃ alcoxy-C₁₋₃ alkyle ;
halogène, C₁₋₄ alkyle, nitrile, amino, hydroxyle, C₁₋₆ alcoxy, NH₂C(O), mono- ou di(C₁₋₃ alkyl)aminocarbonyle, mono- ou di-(C₁₋₃ alkyl)amino, amine secondaire ou tertiaire où l'azote d'amino est lié de manière covalente à C₁₋₃ alkyle ou C₁₋₅ alcoxyalkyle, pyridinyl-C₁₋₃ alkyle, imidazolyl-C₁₋₃ alkyle, tétrahydrofuranyl-C₁₋₃ alkyle, nitrile-C₁₋₃ alkyle, carboxamide-C₁₋₃ alkyle, phényle, où le cycle phényle est éventuellement substitué avec un à deux halogènes, C₁₋₆ alcoxy, hydroxyle ou mono- ou di-(C₁₋₃ alkyl)amino, C₁₋₆ alkyl-S(O)ₘ, ou phényl-S(O)ₘ, où le cycle phényle est éventuellement substitué avec un à deux halogènes, C₁₋₆ alcoxy, hydroxyle, halogène ou mono- ou di-(C₁₋₃ alkyl)amino ;
C₁₋₆ alkyl-S(O)ₘ et phényl-S(O)ₘ, où le cycle phényle est éventuellement substitué avec un à deux halogènes, C₁₋₆ alcoxy, hydroxyle ou mono- ou di-(C₁₋₃ alkyl)amino ;
R₁ et R₂ sont choisis indépendamment parmi :
un C₁₋₆ alkyle ramifié ou non ramifié éventuellement partiellement ou totalement halogéné, C₁₋₄ alcoxy ramifié ou non ramifié, chacun étant éventuellement partiellement ou totalement halogéné, halogène, C₁₋₃ alkyl-S(O)ₘ éventuellement partiellement ou totalement halogéné et phénylsulfonyle ;
m est 0, 1 ou 2 ;
ledit procédé comprenant :
i) la réaction d'un ester Z-Y-X-COO-Rₓ (II) avec un diester (III), où Rₓ dans (II) et (III) est indépendamment C₁₋₅ alkyle ou aryle, dans un solvant approprié et une base appropriée et éventuellement en présence d'un additif à une température de 0 à 200°C pendant une durée de réaction de 5 min à 24 h, et l'isolement du composé intermédiaire (IV) : 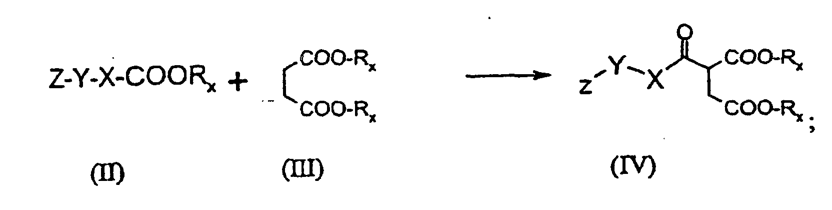
ii) l'hydrolyse du produit de l'étape i) avec hydrolyse acide ou basique, et la décarboxylation dans des conditions acides appropriées à une température d'environ 50 à 200°C et pendant 5 min à 24 heures ; suivie par l'estérification dans des conditions appropriées avec un C₁₋₅alcool ; puis l'addition nucléophile de phényle où le phényle est éventuellement indépendamment substitué par R₁ et R₂ ; le clivage par clivage réducteur dans des conditions appropriées pour former un composé acide carboxylique, et le traitement avec un acide minéral fort éventuellement dans un solvant approprié à TA à 200°C, pour former un produit intermédiaire de formule (V), et l'isolement du produit : 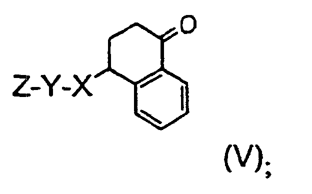
iii) la réaction du produit de l'étape ii) avec HNR_{y}R_{z} où son sel respectif, pour former un composé énamine ou imine de formule (VI) dans des conditions appropriées à TA pendant 1 à 24 h : 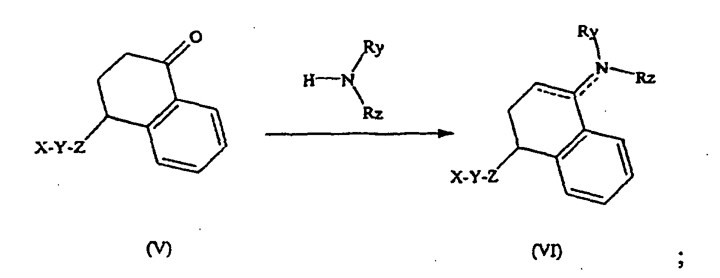 où R_{y} est C₁₋₅ alkyle ou l'hydrogène, R_{z} est C₁₋₅ alkyle, l'hydrogène ou OH avec la condition que, quand la formule (VI) est un tautomère énamine, R_{y} et R_{z} sont l'un et l'autre C₁₋₅ alkyle, ou quand la formule (VI) est un tautomère imine, R_{z} est OH, C₁₋₅ alkyle ou l'hydrogène et R_{y} n'est pas présent : 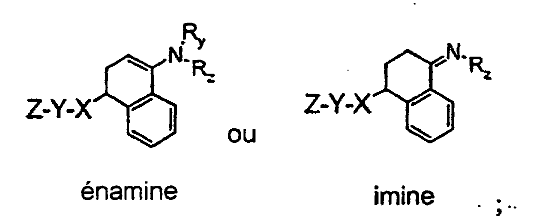
iv) quand le produit de l'étape iii) est une imine, dans une réaction en un récipient, l'acylation et la réduction du produit (VI) ; puis le traitement du produit d'une telle réaction dans des conditions oxydantes capables de former le cycle naphtalène dans la formule (I), suivi par la déprotection dans des conditions appropriées ;
ou
quand le produit de l'étape iii) est une énamine, l'oxydation de l'énamine dans des conditions oxydantes appropriées pour former le cycle naphtalène, puis la déprotection de l'azote d'amino pour former la formule (I) :

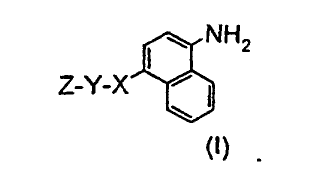

2. Procédé selon la revendication 1 où :
X est choisi parmi
un C₅₋₈ cycloalkyle et cycloalcényle éventuellement substitué avec un ou deux groupes oxo ou une à trois chaînes C₁₋₄ alkyle, C₁₋₄ alcoxy ou C₁₋₄ alkylamino chacune étant ramifiée ou non ramifiée ;
aryle, pyridinyle, pyrimidinyle, pyridinonyle, dihydropyridinonyle, maléimidyle, dihydromaléimidyle, pipéridinyle, benzimidazole, 3H-imidazo[4,5-b]pyridine, pipérazinyle, pyridazinyle et pyrazinyle ; chacun étant éventuellement substitué indépendamment avec un à trois C₁₋₄ alkyle, nitro, nitrile, mono- ou di-(C₁₋₃ alkyl)amino, mono- ou di-(C₁₋₃ alkylamino)carbonyle, NH₂C(O), C₁₋₆ alkyl-S(O)ₘ ou halogène ;
Y est choisi parmi
une liaison et
une chaîne carbonée C₁₋₄ saturée ou insaturée où l'un des atomes de carbone est éventuellement remplacé par O, N ou S(O)ₘ et où Y est éventuellement indépendamment substitué avec un ou deux groupes oxo, phényle ou un ou plusieurs C₁₋₄ alkyle éventuellement substitués par un ou plusieurs atomes d'halogène ;
Z est choisi parmi
phényle, pyridinyle, pyrimidinyle, pyridazinyle, pyrazinyle, imidazolyle, furanyle, thiényle, dihydrothiazolyle, dihydrothiazolyl sulfoxydyle, pyranyle, pyrrolidinyle qui sont éventuellement substitués avec un à trois nitriles, C₁₋₃ alkyle, C₁₋₃ alcoxy, amino, hydroxyle, mono- ou di-(C₁₋₃ alkyl)amino ou CONH₂ ;
tétrahydropyranyle, tétrahydrofuranyle, 1,3-dioxolanonyle, 1,3-dioxanonyle, 1,4-dioxanyle, morpholinyle, thiomorpholinyle, thiomorpholinyl sulfoxydyle, pipéridinyle, pipéridinonyle, pipérazinyle, tétrahydropyrimidonyle, pentaméthylène sulfuryle, pentaméthylène sulfoxydyle, pentaméthylène sulfonyle, tétraméthylène sulfuryle, tétraméthylène sulfoxydyle ou tétraméthylène sulfonyle qui sont éventuellement substitués avec un à trois nitriles, C₁₋₃ alkyle, C₁₋₃ alcoxy, hydroxyle, amino, mono- ou di-(C₁₋₃ alkyl)amino ou CONH₂ ;
nitrile, C₁₋₆ alkyl-S(O)ₘ, halogène, hydroxyle, C₁₋₄ alcoxy, amino, mono- ou di- (C₁₋₆ alkyl)amino, mono- ou di-(C₁₋₃ alkyl)aminocarbonyle et NH₂C(O).

3. Procédé selon la revendication 2 où :
X est
aryle, pyridinyle, pyrimidinyle, benzimidazole, 3H-imidazo[4,5-b]pyridine, pipérazinyle, pyridazinyle et pyrazinyle ; chacun étant éventuellement substitué indépendamment avec un à trois C₁₋₄ alkyle, nitro, nitrile, mono- ou di-(C₁₋₃ alkyl)amino, mono- ou di-(C₁₋₃ alkylamino)carbonyle, NH₂C(O), C₁₋₆ alkyl-S(O)ₘ ou halogène ;
Y est choisi parmi
une liaison et
une chaîne carbonée C₁₋₄ saturée où l'un des atomes de carbone est éventuellement remplacé par O, N ou S et où Y est éventuellement indépendamment substitué avec un groupe oxo ;
Z est choisi parmi
phényle, pyridinyle, pyrimidinyle, pyridazinyle, pyrazinyle, imidazolyle, dihydrothiazolyle, dihydrothiazolyl sulfoxyde, pyranyle et pyrrolidinyle qui sont éventuellement substitués avec un à deux C₁₋₂ alkyle ou C₁₋₂ alcoxy ;
tétrahydropyranyle, morpholinyle, thiomorpholinyle, thiomorpholinyl sulfoxydyle, pipéridinyle, pipéridinonyle, pipérazinyle et tétrahydropyrimidonyle qui sont éventuellement substitués avec un à deux C₁₋₂ alkyle ou C₁₋₂ alcoxy ; et C₁₋₃ alcoxy.

4. Procédé selon la revendication 3 où
X est choisi parmi
pyridinyle et pyrimidinyle, chacun étant éventuellement substitué indépendamment avec un à trois C₁₋₄ alkyle, nitro, nitrile, mono- ou di-(C₁₋₃ alkyl)amino, mono- ou di-(C₁₋₃ alkylamino)carbonyle, NH₂C(O), C₁₋₆ alkyl-S(O)ₘ ou halogène ;
Y est choisi parmi
une liaison, -CH₂-, -CH₂CH₂-, -C(O)-, -O-, -S-, -NH-CH₂CH₂CH₂-, -N(CH₃)- et -NH-.

5. Procédé selon la revendication 4 où :
Y est choisi parmi
-CH₂-, -NH-CH₂CH₂CH₂- et -NH- et
Z est morpholinyle.

6. Procédé selon les revendications 1,2,3,4 ou 5 où :
dans l'étape i) :
Rₓ est C₂H₅ ;
le solvant est aprotique choisi parmi THF, DME, DMSO, l'éther, le dioxane, CH₂Cl₂, CHCl₃, le toluène, la pyridine et DMF ;
la base est choisie parmi NaH, NaNH₂, le t-butylate de Na, NaOH, la pyridine, TEA, l'éthylate de Na, DBU et BuLi ;
l'additif est choisi parmi DMPU et HMPA ;
et la durée de réaction est 8 h.
dans l'étape ii) :
l'hydrolyse est acide ;
les conditions acides de décarboxylation sont H₂SO₄ conc. dans HOAc, la température est 100°C et la durée est 7 h ;
l'estérification est avec CH₃CH₂OH ;
l'addition nucléophile de phényle est par le biais d'un réactif de Grignard phényle PhMgBr ;
le clivage réducteur est avec HCOONH₄/Pd/C/EtOH ;
l'acide minéral ajouté est PPA et à une température de 110°C ;
dans l'étape iii) :
la formation d'une oxime dans des conditions de formation d'oxime par addition de HNR_{y}R_{z} où ledit HNR_{y}R_{z} est le sel d'amine NH₂OH,HCl, dans EtOH avec NaOH pendant 18 h ;
dans l'étape iv) :
le produit de l'étape iii) est acétylé avec l'anhydride acétique et l'acide acétique puis le composé (VI) est réduit avec Fe à 55°C pendant 5 heures ;
les conditions oxydantes sont le traitement du produit avec DDQ dans un solvant non polaire, à 0 à 50°C, à 0,5 à 10 h.

7. Procédé selon la revendication 6 où :
dans l'étape i) :
le solvant est THF ;
la température est 60-70°C ;
la base est choisie parmi NaH et le t-butylate de Na ;
l'additif est DMPU ;
et
dans l'étape iv) :
pour l'étape oxydante le solvant non polaire est le chlorure de méthylène, la température est environ TA et la durée est 5 h.
